# EUROPEAN PATENT SPECIFICATION

(11) **EP 0 636 179 B1**
(45) Date of publication and mention of the grant of the patent: **22.09.2004**
(21) Application number: 92908088.5
(22) Date of filing: 15.04.1992
(51) Int. Cl.: C12N 15/29, C12N 15/62, C12N 15/82, C12P 21/02, C12N 5/10, A01H 5/00

(54) **OIL-BODY PROTEINS AS CARRIERS OF HIGH-VALUE PEPTIDES IN PLANTS**
ÖL-KÖRPER PROTEINE ALS TRÄGER VON HOCHWERTIGEN PEPTIDEN IN PFLANZEN
PROTEINES DE CORPS HUILEUX UTILISEES EN TANT QUE VECTEURS DE PEPTIDES DE GRANDE VALEUR DANS DES PLANTES

(43) Date of publication of application: 01.02.1995
(73) Proprietor: SemBioSys Genetics Inc., Calgary, Alberta T1Y 7L3 (CA)
(72) Inventor: MOLONEY, Maurice, M., Calgary, Alberta T3A 2S4 (CA)
(74) Representative: Harding, Charles Thomas
(86) International application number: PCT/CA1992/000161
(87) International publication number: WO 1993/021320

(56) References cited:
- EMBL Nucleic Acid Sequence Database Accession No. X62353 20-2-1992 Release 31 A. thaliana gene for oleosin
- PLANT MOLECULAR BIOLOGY. vol. 18, no. 6, April 1992, DORDRECHT, THE NETHERLANDS. pages 1177 - 1179 VAN ROOIJEN, G.J.H. 'Nucleotide sequence of an Arabidopsis thaliana oleosin gene'
- PROCEEDINGS OF THE NATIONAL ACADEMY OF SCIENCES OF USA vol. 88, July 1991, WASHINGTON US pages 6181 - 6185 LEE, W.S., ET AL 'Maize oleosin is correctly targeted to seed oil bodies in Brassic napus transformed with the maize oleosin gene'
- J. EXP. BOT., SUPPL., MEETING HELD APRIL 7-12, 1991. vol. 42, no. 238, 1991, page 47 BATCHELDER, C., ET AL. 'Molecular genetics of oil body membrane proteins in Brassica napus and Arabidopsis'
- BIOTECHNOLOGY vol. 7, no. 9, September 1989, NEW YORK US pages 929 - 932 VANDEKERCKHOVE, J., ET AL. 'Enkephalins produced in transgenic plants using modified 2S seed storage proteins'
- PLANT PHYSIOLOGY. vol. 96, 1991, ROCKVILLE, MD, USA. pages 1395 - 1397 LEE, K., ET AL. 'Genomic nucleotide sequence of a Brassica napus 20-kilodalton oleosin gene'
- BIOLOGICAL ABSTRACTS vol. 91 , 1991, Philadelphia, PA, US; abstract no. 84634, MURPHY, D.J., ET AL. 'A class of amphipathic proteins associated with lipid storage bodies in plants possible similarities with animal serum apolipoproteins'
- VAN ROOIJEN ET AL: 'Structural Requirements of Oleosin Domains for subcellular targetting to the oil body' PLANT PHYSIOL. vol. 109, 1995, pages 1353 - 1361
- ABELL ET AL: 'Role of the proline knot motif in oleosin endoplasmic reticulum topology and oil body targetting' THE PLANT CELL vol. 9, August 1997, pages 1481 - 1493
- VAN ROOIJEN & MOLONEY: 'Plant seed oil-bodies as carriers for foreign proteins' BIO/TECHNOLOGY vol. 13, January 1995, pages 72 - 77
- PARMENTER ET AL: 'Production of biologically active hirudin in plat seeds using oleosin partitioning' PLANT MOLECULAR BIOLOGY vol. 29, 1995, pages 1167 - 1180

## Description

### INTRODUCTION

### Technical Field

This invention relates to a method for the production by recombinant means of a protein of interest which is easily purified from host cell components. The method is exemplified by expression of the protein of interest in plants, particularly seeds, as a chimeric peptide comprising an oil-body protein and the protein of interest.

### Background

A variety of proteins have been expressed in plants. However, while the general feasibility of obtaining expression of foreign proteins in plants has been demonstrated, obtaining purified proteins from this source has some limitations. These limitations include the purification step necessary to obtain pure protein essentially free of plant derived materials and the degradation that may occur in extracts prepared during the purification procedure when the recombinant proteins obtained are in contact with aqueous buffers.

Plants bearing oilseeds such as soybean, rapeseed, sunflower and a number of other plant species such as corn, carrot, etc., store triglycerides in their seeds. In the plant, these triglycerides act as a source of energy for a germinating seed and the subsequent seedling. The triglycerides are widely used as vegetable oils in foods and in food preparation and also for some industrial applications.

Triglycerides are immiscible with water and partition by floating on the surface of aqueous solutions or by forming small globules or liposomes as a suspension in the aqueous phase. Such globules will naturally coalesce if they are not stabilized by a modified surface layer. This coalescence can result in a suspension of globules of random sizes. In seeds, when triglyceride is stored, the oil globules are actually encapsulated lipid or oil bodies normally of uniform size. Associated with the surface of these oil bodies is a half unit membrane studded with several proteins, generally referred to as oil-body proteins.

At least one class of oil-body proteins has some characteristics which are highly conserved between species. This class of oil-body proteins is referred to as an "oleosin." The hydrophilic *N-* and C-termini of these proteins appear to be quite divergent, whereas the lipophilic internal region (central core) appears to be highly conserved between species. The oleosins are strongly associated with the oil bodies; this strong association to the oil-bodies may, in major part, be due to the lipophilic nature of these central core. It is therefore of interest to determine whether oil body proteins such as oleosins may be useful in a method for the production of recombinant proteins by providing a means for separation of the recombinant proteins from plant derived materials.

### Relevant Literature

The production of foreign (recombinant) peptides in plants has been investigated using a variety of approaches including transcriptional fusions using a strong constitutive plant promoter (e.g., from cauliflower mosaic virus--Sijmons *et al.* (1990) *Bio*/*Technology, 8*:217-221) and the coding of a foreign protein; transcriptional fusions with organ specific sequences (Radke *et al.* (1988) *Theoret. Appl. Genet., 75*:685-694); and translational fusions which require subsequent cleavage of a recombinant protein (Vander Kerkove *et al.* (1989) *Bio*/*Technology, 7*:929-932). Foreign proteins which have been expressed in plant cells include active proteins from bacteria (Fraley *et al.* (1983) *Proc.* *Nat'l. Acad. Sci. USA, 80*:4803-4807), animals (Misra and Gedamu (1989) *Theor. Appl. Genet., 78*:161-168), fungi and other plant species (Fraley *et al.* (1983) *Proc. Nat'l. Acad. Sci. USA, 80*:4803-4807).

Some proteins, normally markers of integration, have been expressed in a tissue-specific manner, including some in seeds (Sen Gupta-Gopalan *et al.* (1985) *Proc. Nat'l. Acad. Sci. USA, 82*:3320-3324); Radke *et al.* (1988) *Theor. Appl. Genet., 75*:685-694). These reports have concentrated specifically on the use of seed-storage protein promoters as a means of deriving seed-specific expression. Using such a system, Vanderkerkove *et al.* (1989) *Bio*/*Technol., 7*:929-932, expressed a high value peptide (leu-enkephalin) in seeds of *Arabidopsis thaliana* and *Brassica napus.* The yield of this peptide was quite low, but demonstrates the feasibility of expression of an animal peptide hormone in plant tissues. Maize oleosin has been expressed in seed oil bodies in *Brassica napus* transformed with a maize oleosin gene. The gene was expressed under the control of regulatory elements from a *Brassica* gene encoding napin, a major seed storage protein. The temporal regulation and tissue specificity of expression was reported to be correct for a napin gene promotor/terminator. See, Lee *et al.*, *Proc. Nat'l. Acad. Sci.* (USA) (1991) 88:6181-6185.

The oil globules which are produced in seeds all appear to be of a similar size, indicating that they are stabilized (Huang A.H.C. (1985) in *Modern Meths. Plant Analysis,* Vol. 1:145-151 Springer-Verlag, Berlin). On closer inspection, it has been found that these are not simple oil-globules, but rather oil-bodies surrounded by a membrane. These oil-bodies have been variously named by electron microscopists, oleosomes, lipid bodies and spherosomes (Gurr MI. (1980) *in The Biochemistry of Plants,* 4:205-248, Acad. Press, Orlando, Fla). The oil-bodies of a few species have been studied and the general conclusion is that they are encapsulated by an unusual "half-unit" membrane comprising not a classical lipid bilayer, but rather a single amphophilic layer with hydrophobic groups on the inside and hydrophilic groups on the outside (Huang A.H.C. (1985) *in Modern Meths. Plant Analysis,* Vol. *1*:145-151 Springer-verlag, Berlin).

Analysis of the contents of lipid bodies has demonstrated that apart from triglyceride and membranous material, there are also several polypeptides/proteins associated with the surface or lumen of the oil body (Bowman-Vance and Huang (1987) *J*. *Biol. Chem., 262*:11275-11279, Murphy *et al.* (1989) *Biochem. J., 258*:285-293, Taylor *et al.* (1990) *Planta, 181*:18-26). Oil-body proteins have been identified in a wide range of taxonomically diverse species (Moreau *et al.* (1980) *Plant Physiol., 65*:1176-1180; Qu *et al.* (1986) *Biochem. J., 235*:57-65) and been shown to be uniquely localized in oil-bodies and not found in organelles of vegetative tissues. In Brassica napus (rapeseed) there are at least three polypeptides associated with the oil-bodies of developing seeds (Taylor *et al.* (1990), *Planta, 181:*18-26). The numbers and sizes of oil-body associated proteins may vary from species to species. In corn, for example, there are two immunologically distinct polypeptide classes found in oil-bodies (Bowman-Vance and Huang (1988) *J. Biol. Chem., 263:*1476-1481). Oleosins have been shown to comprise regions of alternate hydrophilicity, hydrophobicity and hydrophilicity (Bowman-vance and Huang (1987) *J. Biol. Chem., 262:*11275-11279). The amino acid sequences of oleosins from corn, rapeseed, and carrot have been obtained. See Qu and Huang (1990) *J. Biol. Chem., 265:*2238-2243, Hatzopoulos *et al.* (1990) *Plant Cell, 2*:457-467, respectively. In an oilseed such as rapeseed, oleosin may comprise between 8% (Taylor et al. (1990) *Planta, 181*:18-26) and 20% (Murphy *et al.* (1989) *Biochem. J., 258*:285-293) of total seed protein. Such a level is comparable to that found for many seed storage proteins.

Genes encoding oil-body proteins have been reported for two species, maize *(Zea mays,* Bowman-vance and Huang (1987) *J. Biol. Chem.,* 262:11275-11279; and Qu and Huang (1990) J. Biol. Chem., 265:2238-2243) and carrot (Hatzopoulos *et al.* (1990) *Plant Cell, 2*:457-467).

QU and Huang (1990) *J. Biol. Chem., 265*:2238-2243 teach a maize oleosin, including the sequence of a cDNA clone containing the complete coding sequence as well as the 5' and 3' flanking regions. It is suggested that the amino acid has three major structural domains, a largely hydrophilic domain at the N-terminal end, a hydrophobic hairpin α-helical domain at the centre and an amphipathic α-helix domain at the C-terminus.

### SUMMARY OF THE INVENTION

Methods and compositions are provided for the production of peptides which may be easily purified from host proteins. The method includes the steps of preparing a chimeric DNA construct which includes a sequence encoding an oil-body specific sequence comprising the coding sequence of a seed-specific oil-body protein gene, or a sequence encoding at least a portion of the hydrophobic core of an oil-body protein, and a coding sequence for a peptide of interest from which an expression cassette containing the chimeric DNA construct can be prepared; transforming a host cell with the expression cassette under genomic integration conditions; and growing the resulting transgenic plant to produce seed in which the polypeptide of interest is expressed as a fusion protein with the oleosin.

The polypeptide of interest may be purified by isolating oil-bodies from the cells of the seed, and disrupting the oil-bodies so that the fusion protein is released. The oil-body protein is then easily separated from other proteins and plant derived material by phase separation. Optionally a cleavage site may be located at least one of the prior to the N-terminus and after the C-terminus of the polypeptide of interest allowing the fusion polypeptide to be cleaved and separated by phase separation into its component peptides. The production system thus provides for targeting of the chimeric peptide by its oil-body protein functionality to the oil bodies which, in turn, permits rapid purification of the polypeptide of interest. This production system finds utility in the production of many peptides such as those with pharmaceutical, enzymic, rheological and adhesive properties.

In accordance with the present invention there is provided a chimeric DNA construct encoding a chimeric polypeptide and comprising:
(a) a first DNA sequence encoding at least the central hydrophobic core of an oil body protein and sufficient to provide for targeting of the fusion polypeptide to an oil body and
(b) a second DNA sequence encoding a peptide
wherein said peptide is other than a polypeptide naturally present in an oil body.

The present invention also provides an expression cassette comprising as components, in the direction of transcription:
- a regulatory DNA sequence comprising a sufficient portion of the region 5' to the translational start site of a gene expressed in seed to provide for expression of a DNA sequence in seed;
- a chimeric DNA sequence encoding a chimeric polypeptide and comprising:
   (a) a first DNA sequence encoding at least the central hydrophobic region of an oil body protein and sufficient to provide for targeting of the chimeric polypeptide to an oil body, said first DNA sequence including at least one restriction site, and
   (b) a second DNA sequence encoding a peptide wherein said peptide is other than a polypeptide naturally present in an oil body; and
- a translational and transcriptional termination region;
wherein said components are operably linked and expressed by said chimeric DNA sequence which is regulated by said regulatory DNA sequence.

The present invention yet also provides a method for obtaining expression of a peptide of interest in seed, said method comprising:
transforming a host plant cell with an expression cassette according to the present invention or a DNA construct according to the present invention under genomic integration conditions.

The present invention also provides a seed, a plant or a plant cell comprising an expression cassette according to the present invention or a DNA construct according to the present invention. The present invention also provides a chimeric polypeptide comprising at least the central hydrophobic core of an oil body protein and sufficient to provide for targeting to an oil body and a second polypeptide, said chimeric polypeptide being expressed by the expression cassette according to the present invention or a chimeric DNA construct according to the present invention.

### BRIEF DESCRIPTION OF THE DRAWINGS

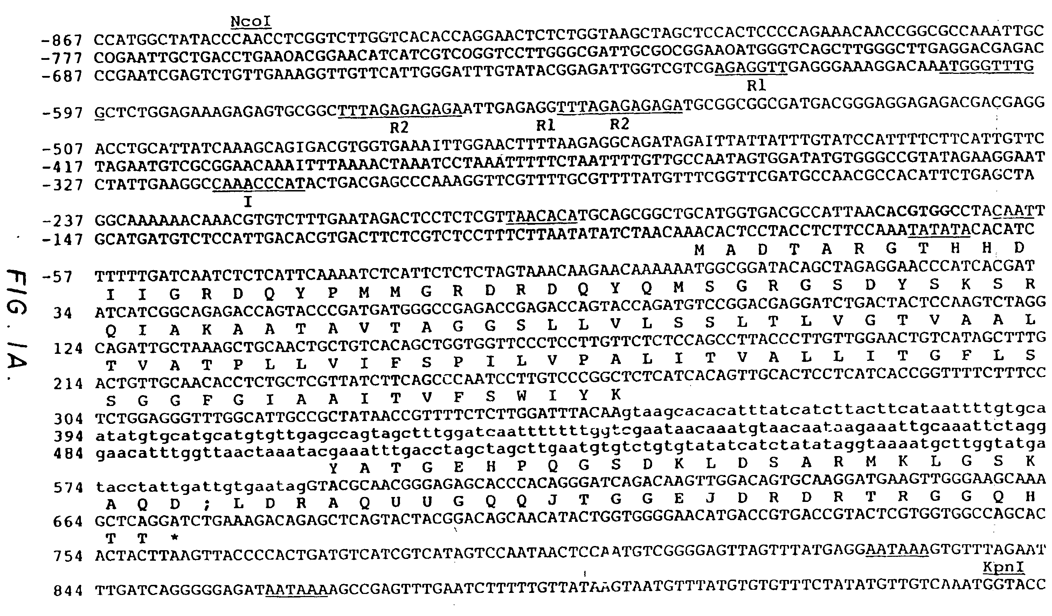

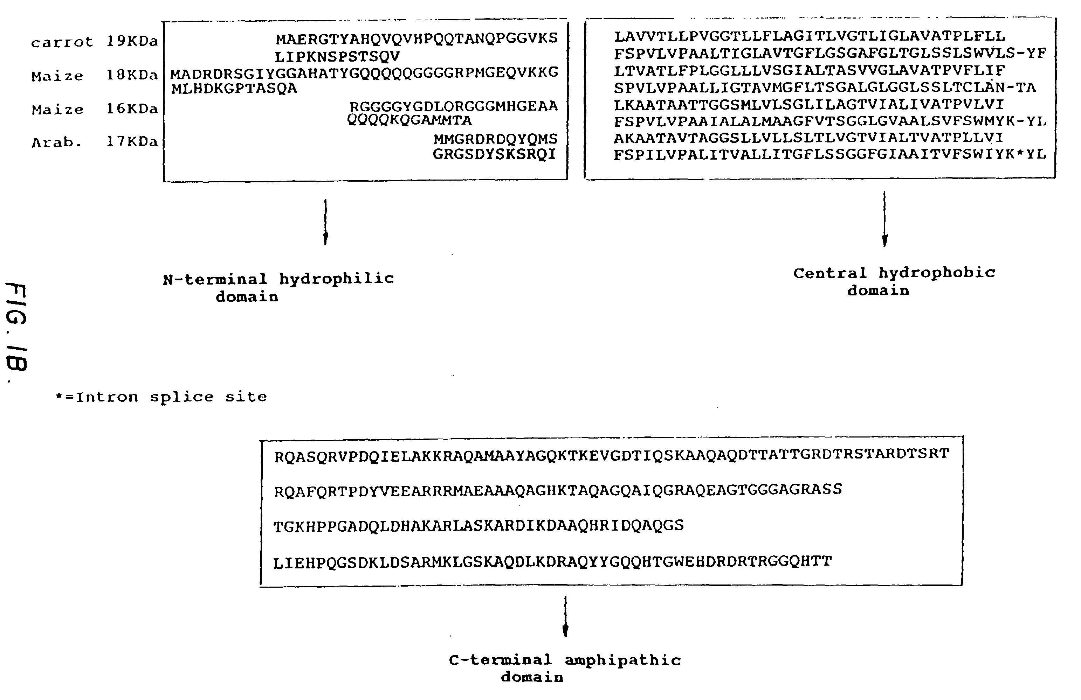

Fig. 1A. shows the nucleotide sequence and deduced amino-acid sequence (17 kDa protein) of an oil-body protein gene (oleosin) from *Arabidopsis thaliana.* Underlined are the direct repeats (R1 and R2) and inverted repeat (T), a CACA, TATA, TAAT and polyadenylation signals. The intron sequence is printed in lower case and a putative ABA-binding site is indicated in bold.
Fig. 1B. shows a comparison of the sequences of oil-body 16 Kd protein from carrot, an 18 Kd and 16 Kd oil-body protein from maize and a 17 Kd oil-body protein from *Arabidopsis thaliana* indicating conserved and divergent regions of the proteins; the amino acid sequences are aligned to show the conservation of sequence in the central region of the proteins.

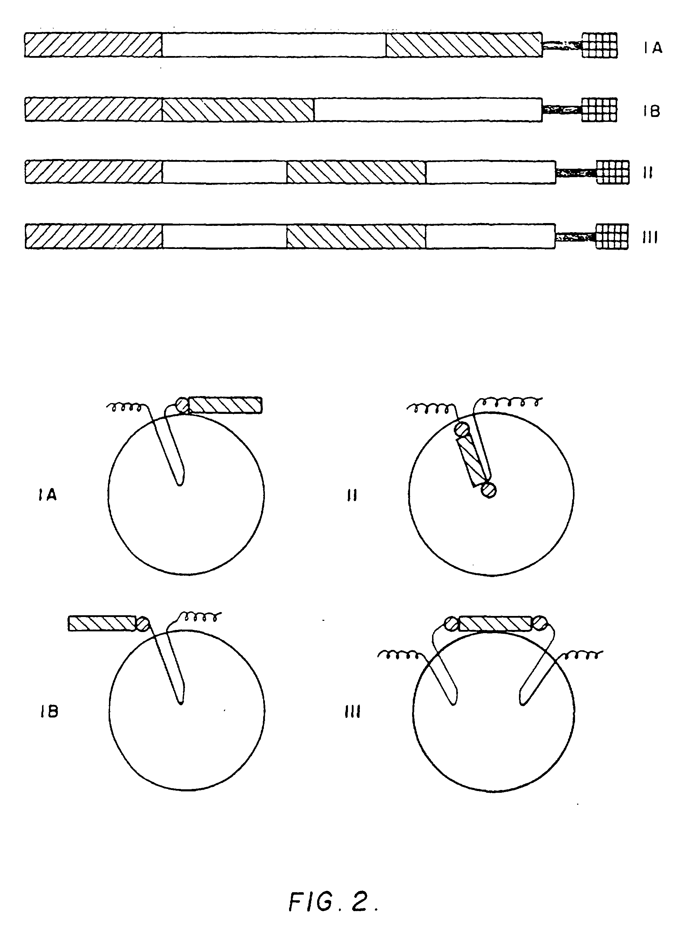

Fig. 2. shows constructs used for the fusion of oil-body protein genes with genes encoding foreign peptides. IA is a C-terminal fusion of a desired peptide to OBP; IB is an N-terminal fusion of a desired peptide to OBP; II is an internal fusion of a desired peptide within OBP; and III is an inter-dimer translational fusion of desired peptide enclosed between two substantially complete oil body protein targeting sequences. In the upper portion of Figure (A) are shown the DNA constructs used for translational fusions of desired peptides to oil-body proteins. In the lower portion of Figure (B) are shown the configurations of the gene products, shown on the upper portion of the translation and the delivery to the oil bodies. The key to the figure is as follows: bottom left-top right hatched box represents an OBP promoter or other seed specific promoter; bottom right-top left hatched box represents a desired peptide coding sequence; open box represents an oil-body protein coding sequence or synthetic targeting sequence based on OBP conserved motifs; vertical-horizontal hatched box represents a gene terminator containing a polyadenylation signal; hatched circle represents a protease recognition motif; corkscrew line represents a native C- or N-terminal of OBP.

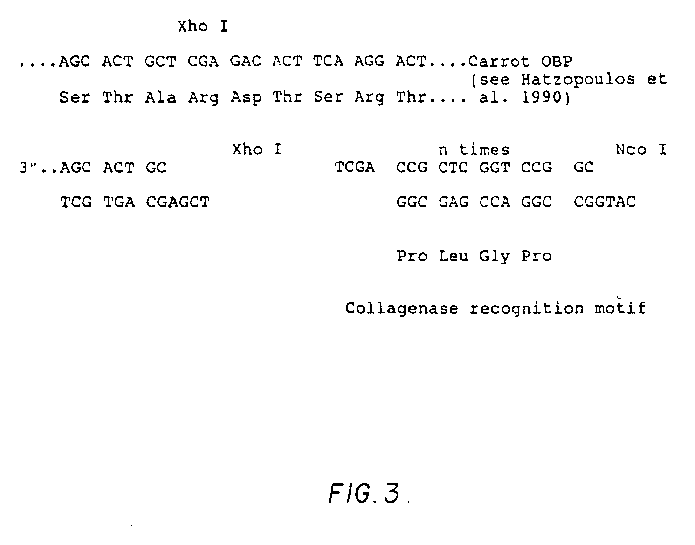

Fig. 3. shows a detailed arrangement for construction of a C-terminal fusion. Shown is the arrangement is a collagenase recognition motif coding sequence as a linker in the fusion of a typical oil-body protein gene and a fusion peptide, to be linked here using an NcoI, for cloning and expression in plants.

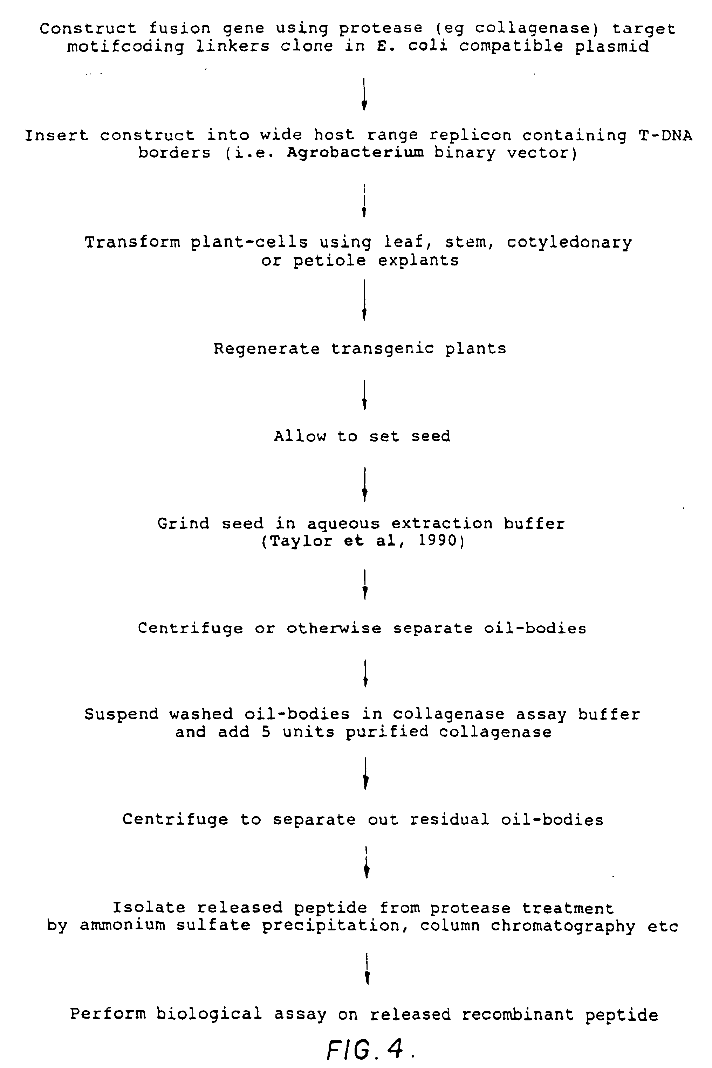

Fig. 4. shows schematically the process of construction of fusion peptide vectors, their introduction into plants and subsequent extraction and assay of the desired recombinant peptide.

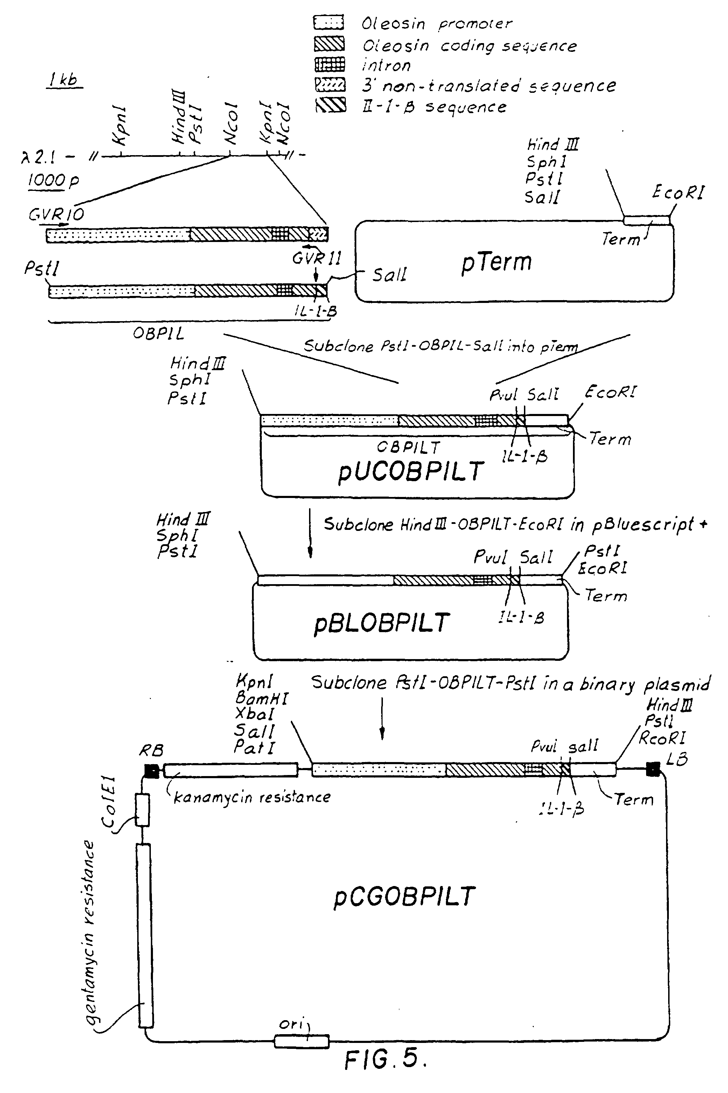

Figure 5 shows a schematic representation of the construction of pCGOBPILT. The broken line box represents an oleosin promoter; the top left-bottom right hatched box represents an oleosin coding sequence; the horizontal-vertical hatched box represents an intron; the dotted box represents a 3' non-translated sequence; and the widely-spaced top left-bottom right hatched box represents an interleukin-1-β sequence equipped with a sequence encoding a protease cleavage site (Factor Xa or thrombin immediately upstream).

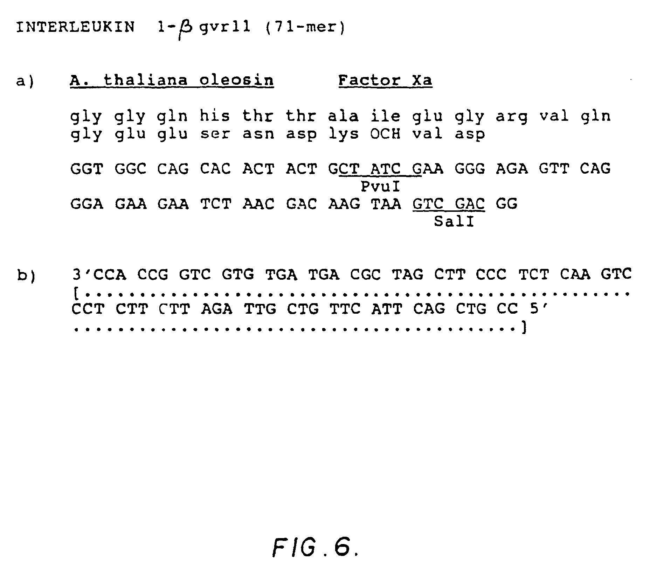

Figure 6 shows the design of oligonucleotide GVR11. In Figure 3A represents the 3' coding sequence of the *A. thaliana oleosin,* translationally fused to the factor Xa/IL-1-β coding sequence followed by a TAA stop codon. For future cloning purposes, a *Pvu*I and *Sal*I restriction enzyme recognition site are included. The creation of a *Pvu*I restriction site resulted in the additional coding sequence for an alanine (ala). Underlined are the restriction enzyme recognition sequences. Overlined are the *A. thaliana* oleosin sequences and the factor Xa recognition sequence. The actual cleavage site is indicated with an asterisk (*). In Figure 3B, the sequence of GVR11 is shown. In order to make a fusion with the *A. thaliana* oleosin, the primer GVR11 needs to be a sequence complementary to the top strand.

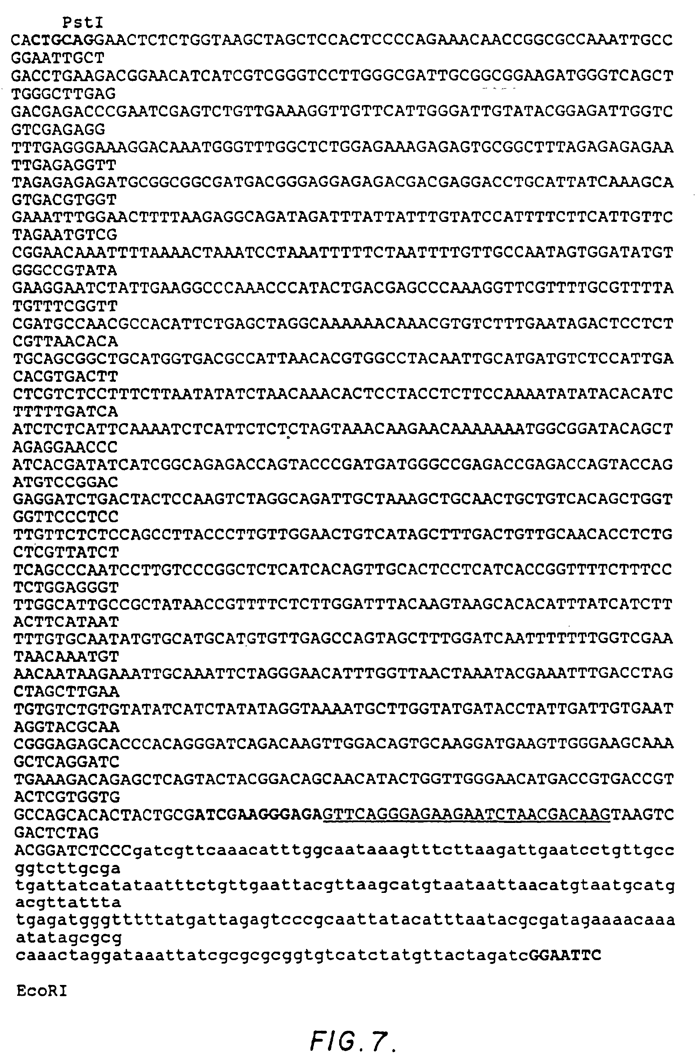

Figure 7 shows the nucleotide sequence of OBPILT. Underlined is the sequence encoding IL-1-β; the sequence encoding the factor Xa recognition site is indicated in bold. The nopaline synthase terminator sequence is indicated in lower case letters.

### DESCRIPTION OF THE SPECIFIC EMBODIMENTS

In accordance with the subject invention, methods and compositions are provided for production of peptides which are easily purified. The subject method includes the steps of preparing an expression cassette containing DNA sequences encoding a sufficient portion of an oil body specific sequence, such as an oleosin, to provide for targeting to an oil body and the peptide of interest; transforming the expression cassette into a plant cell host; generating a transgenic plant and growing it to produce seed in which the chimeric protein is expressed and translocated to the oil bodies. The chimeric peptide comprises the peptide of interest and an oil body protein such as an oleosin. The peptide of interest generally is a foreign peptide normally not expressed in seeds or found on the oil-body. The use of an oil-body protein as a carrier or targeting means provides a simple mechanism to obtain purification of the foreign protein. The chimeric protein is separated away from the bulk of cellular protein in a single step (such as centrifugation or flotation); the protein is also protected from degradation during extraction as the separation also removes non-specific proteases from contact with the oil-bodies. The gene encoding the foreign peptide may be derived from any source, including plant, bacterial, fungal or animal source. Desirably, the chimeric peptide will contain sequences which allow for cleavage of the peptide of interest from the oleosin. The method may be employed to express a variety of peptides which are then easily purified.

Targeting a foreign, recombinant protein to the oil-body imparts several advantages, including the following. The protein can be separated from the bulk of cellular contents after cell lysis by centrifugation. The oil-body fraction will float on the surface of the extract. The protein can optionally be provided with a peptide linker containing a protease recognition site. This permits release of the peptide from the oil-body. The protein can be introduced into a recombinant polypeptide in such a way that it is within a lipophilic conserved region. This results in the internalization of the recombinant peptide into the oil-body, thus protecting it from protease attack.

The expression cassette generally will include in the 5'-3' direction of transcription, a transcriptional and translational regulatory region capable of expression in developing seed, typified by the promoter and upstream regions associated with an oil body protein, which will provide for expression of the chimeric protein in seed, a DNA sequence encoding a chimeric peptide comprising an amino acid sequence to provide an oil body targeting means and a protein of interest, and a transcriptional and translational termination region functional in plants. One or more introns may also be present.

The oil-body specific sequence finds analogy in fragments of oil-body proteins, particularly oleosins. The oil-body specific sequence may be the same as that of a sequence obtainable from an oil-body protein, are which has sufficient homology to provide for the desired targeting of a protein of interest to an oil body. By "obtainable" is intended an amino acid sequence which may be natural, synthetic or a combination, sufficiently similar to a native oil body protein amino acid sequence to provide the desired targeting. Of particular interest is the central hydrophobic domain of oil body proteins which appears to be highly conserved among different plant species, and fragments thereof and homologous sequences at the amino acid level.

The deduced amino acid sequence for an *Arabidopsis thaliana* oil-body protein is as fellows: 
10                    20
M-M-G-R-D-R-D-Q-Y-Q-M-S-G-R-G-S-D-Y-S-K-
30                    40
S-R-Q-I-A-K-A-A-T-A-V-T-A-G-G-S-L-L-V-L-
50                    60
S-S-L-T-L-V-G-T-V-I-A-L-T-V-A-T-P-L-L-V-
70                    80
I-F -S-P -I-L-V-P-A-L-I-T-V-A-L-L-I-T-G-F-
90                    100
L-S-S-G-G-F-G-I-A-A-I-T-V-F-S-W-I-Y-K*Y-
110                    120
A -T -G -E-H-P-Q-G-S-D-K-L-D-S-A-R-M-K-L-G-
130                    140
S-K-A-Q-D-L-K-D-R-A-Q-Y-Y-G-Q-Q-H-T-G-G-
150
E-H-D-R-D-R-T-R-G-G-Q-H-T-T
 Amino acids from about 25-101 comprise the central hydrophobic domain.

Of particular interest as a targeting means for some applications are oil-body specific sequences or fragments thereof of the following formula which provide for targeting to an oil body: 
pp¹- aa²⁵- aa²⁶- V - V- T - L- aa³¹- P-
A A A T

aa³⁴- G- G- aa36 - L- L- aa³⁹- L- aa⁴¹-
M

G - I - aa⁴⁴- L- aa⁴⁶- aa⁴⁷- T - L - I -
S L S V V

aa⁵¹- L- aa⁵³- V- A- T- P- L - aa⁵⁹ - L -
V V

L - F- S- P- V - L- V- P- A- A - L - aa⁷³ -
I I L I
 
aa^74 - aa^75 - aa^76 - aa^77 - aa^78 - G- F- L-

G          L
S -  S- aa^87 - G- V - aa^89 - aa^90 - L - S -
T          I                    I   T

aa^93  aa^94 - S - aa^96 - aa^97 - aa^98 - aa^99 -
               T

aa^100 - aa^101 - pp^2
 wherein:
PP¹ and PP² are the same or different, and may be the same as or different from a natural oil-body protein, usually different; they may be hydrogens, indicating the terminal portion of the indicated polypeptide or may be polypeptides having a total of up to 1000 amino acids, more usually of up to about 500 amino acids, and may have a total of as few as 1 amino acid, or may individually or separately be polypeptides of from 1-100 amino acids, more usually from about 1-75 amino acids, more particularly from about 5-50 amino acids; these polypeptides will have specific applications in modifying a specifically described sequence for a predetermined purpose;
aa²⁵ may be any amino acid, particularly a neutral aliphatic amino acid, generally of 3-6 carbon atoms, more particularly leucine or alanine;
aa²⁶ is a neutral aliphatic amino acid, particularly alanine or an hydroxy substituted amino acid of from 3-4 carbon atoms, particularly threonine or a basic amino acid of from 5-6 carbon atoms, particularly lysine;
aa³¹ is a neutral unsubstituted aliphatic amino acid of from 3-6 carbon atoms, particularly alanine, valine or leucine or an aromatic unsubstituted amino acid, particularly phenylalanine;
aa³³ is a neutral unsubstituted aliphatic amino acid of from 3-6 carbon atoms, particularly alanine, valine or leucine or an oxy-substituted aliphatic amino acid, particularly threonine;
aa³⁶ is a neutral aliphatic unsubstituted amino acid of from 3-5 carbon atoms, particularly leucine or a neutral aliphatic oxy-substituted amino acid of from 3-4 carbon atoms, particularly threonine or serine;
aa³⁷ is a neutral unsubstituted amino acid, particularly leucine or a thio-substituted amino acid, particularly methionine;
aa³⁹ is a neutral aliphatic unsubstituted amino acid, particularly valine or an aromatic unsubstituted amino acid, particularly phenylalanine;
aa¹ is a neutral aliphatic unsubstituted or oxy-substituted amino acid, particularly alanine, leucine or serine;
aa⁴⁴ is a neutral aliphatic unsubstituted or oxy-substituted amino acid, particularly alanine, isoleucine or threonine;
aa⁴⁶ is a neutral aliphatic unsubstituted amino acid or an oxy-substituted amino acid, particularly alanine, valine or threonine;
aa⁴⁷ is a neutral aliphatic unsubstituted amino acid, particularly glycine or alanine;
aa⁵⁹ is a neutral aliphatic or aromatic unsubstituted amino acid, particularly leucine or phenylalanine;
aa⁷⁶ is a neutral aliphatic unsubstituted or thio-substituted amino acid, particularly alanine, leucine or methionine;
aa⁷⁸ is a neutral aliphatic unsubstituted amino acid, particularly alanine or a neutral aliphatic amino acid having a thio- or an oxy-substitution, particularly methionine or threonine;
aa⁸³ is a neutral aliphatic unsubstituted or oxy-substituted amino acid, particularly glycine, serine or threonine;
aa⁹² is a neutral aliphatic amino acid with a oxy-substitution, particularly serine or threonine;
aa⁹⁶ is a neutral aliphatic thio-substituted amino acid or a neutral aromatic heterocyclic amino acid, particularly tryptophan;
aa⁹⁷ is a neutral aliphatic unsubstituted or thio-substituted amino acid, particularly valine, leucine, isoleucine or methionine;
aa⁹⁸ is a neutral aliphatic unsubstituted amino acid or an aromatic oxy-substituted amino acid, particularly alanine, leucine or tyrosine;
aa⁹⁹ may be any amino acid;
aa¹⁰⁰ is an oxy-substituted amino acid, either aliphatic or aromatic, particularly tyrosine or threonine;
aa¹⁰¹ is a neutral unsubstituted aliphatic or aromatic amino acid, particularly alanine, leucine or phenylalanine.

Of particular interest as a source of DNA encoding sequences capable of providing for targeting to an oil body protein are oil-body protein genes obtainable from *Arabidopsis* or *Brassica napus* which provide for expression of the protein of interest in seed (See Taylor *et al.* (1990) *Planta, 181*:18-26). The necessary regions and amino-acid sequences to provide targeting ability to the oil body appear to be the highly hydrophobic central region of oil body proteins.

To identify other oil body protein genes having the desired characteristics, where an oil body protein has been or is isolated, the protein may be partially sequenced, so that a probe may be designed for identifying mRNA. Such a probe is particularly valuable if it is designed to target the coding region of the central hydrophobic domain which is highly conserved among diverse species of plants. In consequence, a DNA or RNA probe for this region may be particularly useful for identifying coding sequences of oil body proteins from other plant species. To further enhance the concentration of the mRNA, cDNA may be prepared and the cDNA subtracted with mRNA or cDNA from non-oil body producing cells. The residual cDNA may then be used for probing the genome for complementary sequences, using an appropriate library prepared from plant cells. Sequences which hybridize the cDNA under stringent conditions may then be isolated.

In some instances, as described above, using an oil body protein gene probe (conserved region), a probe may be employed directly for screening a cDNA genomic library and identifying sequences which hybridize to the probe. The isolation may also be performed by a standard immunological screening technique of a seed-specific cDNA expression library. Antibodies may be obtained readily for oil-body proteins using the purification procedure and antibody preparation protocol described by Taylor *et al.* (*Planta*, (1990) *181*, 18-26). cDNA expression library screening using antibodies is performed essentially using the techniques of Huynh et al. (1985, in *DNA Cloning, Vol. 1, a Practical Approach,* ed. D.M. Glover, IRL Press, pp. 49-78). Confirmation of sequence is facilitated by the high conservation found in the central hydrophobic region (see Fig. 1). DNA sequencing by the method of Sanger *et al. (Proc. Natl. Acad. Sci. USA,* (1977) *74*:5463-5467) or Maxam and Gilbert (1980, *Meth. Enzymol.,* (1980) *65*:497-560) may be performed on all putative clones and homology searches performed. Homology of sequences encoding the central hydrophobic domain is normally ≥ 70%, both at the amino-acid and nucleotide level between diverse species. If an antibody is available, confirmation of sequence identity may also be performed by hybrid-select and translation experiments from seed mRNA preparations as described by Sambrook *et al. (Molecular Cloning,* (1990) 2nd Ed., Cold Spring Harbor Press, pp. 8-49 to 8-51).

cDNA clones made from seed can be screened using cDNA probes made from the conserved coding regions of any available oil body protein gene (e.g., Bowman-Vance and Huang *(J. Biol. Chem.,* (1987) *262*:11275-11279). Clones are selected which have more intense hybridization with seed DNAs as compared to seedling cDNAs. The screening is repeated to identify a particular cDNA associated with oil bodies of developing seeds using direct antibody screening or hybrid-select and translation. The mRNA complementary to the specific cDNA is absent in other tissues which are tested. The cDNA is then used for screening a genomic library and a fragment selected which hybridizes to the subject cDNA.

To obtain expression of the chimeric gene in seed a transcriptional initiation regulatory region and translational initiation regulatory region of untranslated 5' sequences, "ribosome binding sites", responsible for binding mRNA to ribosomes and translational initiation obtainable from any gene preferentially expressed in seed may be used. Examples of such genes include seed storage proteins such as from napin (Josefsson *et al., J. Biol. Chem.,* (1987) *262*:12196-12201; Scofield S.R. and Crouch M.L. *J. Biol. Chem.* (1987) *262*:12202-12208). Preferably, the region is obtainable from an oil body protein (oil-body proteins from *Arabidopsis*, carrot (Hatzopoulos *et al.,* supra)or maize (Huang *et al.* 1987 and 1990 supra). The region generally comprises at least 100 bp 5' to the translational start of the structural gene coding sequence, up to 2.5 kb 5' to the same translational start. It is preferred that all of the transcriptional and translational functional elements of the initiation control region are derived from or obtainable from the same gene. By "obtainable" is intended a DNA sequence sufficiently similar to that of a native sequence to provide for the desired specificity of transcription of the DNA sequence encoding the chimeric protein. It includes natural and synthetic sequences and may be a combination of synthetic and natural sequences.

The transcription level should be sufficient to provide an amount of RNA capable of resulting in a modified seed. By "modified seed" is meant seed having a detectably different phenotype from a seed of a non-transformed plant of the same species, for example one not having the expression cassette in question in its genome. various changes in phenotype are of interest. These changes include over-expression of oil-body protein or OBP-accumulation on the oil body or in the cytoplasm of the resultant chimeric protein.

The polypeptide of interest may be any protein and includes for example, an enzyme, an anticoagulant, a neuropeptide, a hormone, or adhesive precursor. Examples of proteins include interleukin-1-β, the anticoagulant Hirudin, the enzyme β-glucuronidase or a single-chain antibody comprising a translational fusion of the V_{H} or V_{L} chains of an immunoglobin. The DNA sequence encoding the polypeptide of interest may be synthetic, naturally derived, or combinations thereof. Depending upon the nature or source of the DNA encoding the polypeptide of interest, it may be desirable to synthesize the DNA sequence with plant preferred codons. The plant preferred codons may be determined from the codons of highest frequency in the proteins expressed in the largest amount in the particular plant species of interest as a host plant.

The termination region which is employed will be primarily one of convenience, since in many cases termination regions appear to be relatively interchangeable. The termination region may be native with the transcrip-tional initiation region, may be native with the DNA sequence encoding the polypeptide of interest, or may be derived from another source. Convenient termination regions are available from the Ti-plasmid of *A. tumefaciens,* such as the octopine synthase and nopaline synthase termination regions.

Ligation of the DNA sequence encoding the targeting sequence to the gene encoding the peptide of interest may take place in various ways including terminal fusions, internal fusions, and polymeric fusions. In all cases, the fusions are made so as not to interrupt the reading frame of the oil-body protein and so as to avoid any translational stop signals in or near the junctions. The different types of terminal and internal fusions are shown in Fig. 2 along with a representation of their configurations *in vivo*.

In all the cases described, the ligation of the gene encoding the peptide preferably would include a linker encoding a protease target motif. This would permit the release of the peptide once extracted as a fusion protein. Potential cleavage sites which could be employed are recognition motifs for thrombin (leu-val-pro-arg-gly) (Fujikawa *et al*., *Biochemistry* (1972) *11*:4892-4899), of factor Xa (phe-glu-gly-arg-aa.) (Nagai *et al., Proc. Nat'l Acad. Sci. USA,* (1985) *82*:7252-7255) or collagenase (pro-leu-gly-pro) (Scholtissek and Grosse *Gene* (1988) 62:55-64).

By appropriate manipulations, such as restriction, chewing back or filling in overhangs to provide blunt ends, ligation of linkers, or the like, complementary ends of the fragments can be provided for joining and ligation. In carrying out the various steps, cloning is employed, so as to amplify the amount of DNA and to allow for analyzing the DNA to ensure that the operations have occurred in proper manner. A wide variety of cloning vectors are available, where the cloning vector includes a replication system functional in E. coli and a marker which allows for selection of the transformed cells. Illustrative vectors include pBR332, pUC series, M13mp series, pACYC184, etc. Thus, the sequence may be inserted into the vector at an appropriate restriction site(s), the resulting plasmid used to transform the *E. coli* host, the *E. coli* grown in an appropriate nutrient medium and the cells harvested and lysed and the plasmid recovered. Analysis may involve sequence analysis, restriction analysis, electrophoresis, or the like. After each manipulation the DNA sequence to be used in the final construct may be restricted and joined to the next sequence, where each of the partial constructs may be cloned in the same or different plasmids.

A variety of techniques are available for the introduction of DNA into plant cell host. For example, the chimeric DNA constructs may be introduced into host cells obtained from dicotyledenous plants, such as tobacco, and oleaginous species, such as Brassica napus using standard Agrobacterium vectors by a transformation protocol such as that described by Moloney *et al. Plant Cell Rep.,* (1989) *8*:238-242 or Hinchee *et al. Bio*/*Technol.*, (1988) *6*:915-922; or other techniques known to those skilled in the art. For example, the use of T-DNA for transformation of plant cells has received extensive study and is amply described in EPA Serial No. 120,516; Hoekema, In: The Binary Plant Vector System Offset-drukkerij Kanters B.V., Alblasserdam, 1985, Chapter V, Knauf, *et al., Genetic Analysis of Host Range Expression by Agrobacterium,* In: Molecular Genetics of the Bacteria. Plant interaction, Puhler, A. ed., Springer-Verlag, NY, 1983, p. 245, and An *et al., EMBO J.* (1985), 4:277-284. Conveniently, explants may be cultivated with *A. tumefaciens* or *A. rhizogenes* to allow for transfer of the transcription construct to the plant cells. Following transformation using Agrobacteria the plant cells are dispersed in an appropriate selective medium for selection, grown to callus, shoots grown and plantlets regenerated from the callus by growing in rooting medium. The *Agrobacterium* host will contain a plasmid having the *vir* genes necessary for transfer of the T-DNA to the plant cells and may or may not have T-DNA. For injection and electroporation, (see below) disarmed Ti-plasmids (lacking the tumor genes, particularly the T-DNA region) may be introduced into the plant cell.

The use of non-Agrobacterium techniques permits the use of the constructs described herein to obtain transformation and expression in a wide variety of monocotyledonous and dicotyledonous plants. These techniques are especially useful for species that are intractable in an Agrobacterium transformation system. Other techniques for gene transfer include biolistics (Sanford, *Trends in Biotech.* (1988) 6:299-302), electroporation (Fromm *et al.* (1985) *Proc. Nat'l. Acad. Sci. USA, 82*:5824-5828; Riggs and Bates (1986), *Proc. Nat'l. Acad. Sci. (USA)* 83 5602-5606 or PEG-mediated DNA uptake (Potrykus *et al.* (1985) *Mol. Gen. Genet., 199*:169-177).

As a host cell, cells from any of a number of seed bearing plants may be employed in which the cells are derived from plant parts such as stem, leaf, root, or seed or reproductive structures according to the species. The cells may be isolated cells or plant parts, for example, leaf discs. In a specific application, such as to *Brassica napus*, the host cells generally will be derived from cotyledonary petioles as described by Moloney *et al. Plant Cell Rep.,* (1989) *8*:238-242). Other examples using commercial oil seeds include cotyledon transformation in soybean explants (Hinchee *et al. Biotechnology,* (1988) *6*:915-922) and stem transformation of cotton (Umbeck *et al. Biotechnology,* (1981) *5*:263-266).

Following transformation, the cells, for example as leaf discs, are grown in selective medium. Once shoots begin to emerge, they are excised and placed onto rooting medium. After sufficient roots have formed, the plants are transferred to soil. Putative transformed plants are then tested for presence of a marker. Southern blotting is performed on genomic DNA using an appropriate probe, for example an *A. thaliana* oleosin gene, to show that integration of the desired sequences into the host cell genome has occurred.

The expression cassette will normally be joined to a marker for selection in plant cells. Conveniently, the marker may be resistance to a herbicide, particularly an antibiotic, such as kanamycin, G418, bleomycin, hygromycin, chloramphenicol, or the like. The particular marker employed will be one which will allow for selection of transformed cells as compared to cells lacking the DNA which has been introduced.

The fusion peptide in the expression cassette constructed as described above, expresses at least preferentially in developing seeds. Accordingly, transformed plants grown in accordance with conventional ways, are allowed to set seed. See, for example, McCormick *et al.* Plant Cell Reports (1986) 5:81-84. Northern blotting can be carried out using an appropriate gene probe with RNA isolated from tissue in which transcription is expected to occur such as a seed embryo. The size of the transcripts can then be compared with the predicted size for the fusion protein transcript.

Oil-body proteins are then isolated from the seed and analyses performed to determine that the fusion peptide has been expressed. Analyses can be for example by PAGE. The fusion peptide can be detected using an antibody to the oleosin portion of the fusion peptide. The size of the fusion peptide obtained can then be compared with predicted size of the fusion protein.

Two or more generations of transgene plants may be grown and either pollinated with the same transformed strain or different strains, identifying the resulting hybrid having the desired phenotypic characteristic, to ensure that the subject phenotypic characteristic is stably maintained and inherited and then seeds harvested for isolation of the peptide of interest or for use to provide seeds with the new phenotypic property.

The desired protein can be extracted from seed that is homo- or heterozygous for the introduced trait by a variety of techniques, including use of an aqueous, buffered extraction medium and a means of grinding, breaking, pulverizing or otherwise disrupting the cells of the seeds. The extracted seeds can then be separated (for example, by centrifugation or sedimentation of the brei) into three fractions: a sediment or insoluble pellet, an aqueous supernatant, and a buoyant "scum" comprising seed storage lipid and oil bodies. These oil bodies contain both native oil-body proteins and chimeric oil body proteins, the latter containing the foreign peptide. The oil-bodies are separated from the water-soluble proteins and re-suspended in aqueous buffer.

If a linker comprising a protease recognition motif has been included in the expression cassette, to the resuspension buffer is added a protease specific for the recognition motif produced by translation of the linker sequence. This releases the required peptide into the aqueous phase. A second centrifugation step will now re-float the processed oil-bodies with their attached proteins and leave an aqueous solution of the required peptide. The desired peptide may be precipitated, chemically modified or lyophilized according to its properties and desired applications

In certain applications it may not be necessary to remove the chimeric protein from the oil-body protein. Such an application would include cases where the fusion peptide includes an enzyme which is tolerant to N or C-terminal fusions and retains its activity; such enzymes could be used without further cleavage and purification. The chimeric enzyme-OBP would be contacted with substrate as a fusion protein. It is also possible, if desired, to purify the enzyme - OBP fusion protein using an immunoaffinity column comprising an immobilized high titre antibody against the OBP (see, for example, Taylor *et al.*, (1990) supra).

Other uses for the subject invention are as follows. OBP's comprise a high percentage of total seed protein, thus it is possible to enrich the seed for certain desirable properties such as high-lysine, high methionine, and the like, simply by making the fusion protein rich in the amino-acid(s) of interest could find utility of particular interest is the modification of grains and cereals which are used as either directly or indirectly as food sources for livestock, including cattle, poultry, and humans. It may be possible to include, as the fusion peptide, an enzyme which may assist in subsequent processing of the oil or meal in conventional oilseed crushing and extraction, for example inclusion of a thermostable lipid-modifying enzyme which would remain active at the elevated crushing temperatures used to process seed and thus add value to the extracted triglyceride or protein product. Other uses of the fusion protein to include use to improve the agronomic health of the crop. For example, an insecticidal protein or a portion of an immunoglobulin specific for an agronomic pest such as a fungal cell wall or membrane, could be coupled to the oil body protein thus reducing attack of the seed by a particular plant pest.

The following examples are offered by way of illustration and not by limitation.

### EXPERIMENTAL

### Example 1

### Expression of terminal fusions of foreign peptides with oil-body proteins

### A. C-terminal fusions

A genomic clone of an oil-body protein gene containing at least 100 bp 5' to the translational start is cloned into a plasmid vehicle capable of replication in a suitable bacterial host (e.g., pUC or pBR322 in *E. coli*). A restriction site is located in the region encoding the hydrophilic C-terminal portion of gene. In a 19 kDa OBP, this region stretches typically from codons 125 to the end of the clone. The ideal restriction site is unique, but this is not absolutely essential. If no convenient restriction site is located in this region, one may be introduced by the site-directed mutagenesis procedure of Kunkel *Proc. Nat'l. Acad. Sci. USA,* (1985) *82*:488-492. The only major restriction on the introduction of this site is that it must be placed 5' to the translational stop signal of the OBP clone.

With this mutated clone in place, a synthetic oligonucleotide adapter may be produced which contains coding sequence for a protease recognition site such as Pro-Leu-Gly-Pro or a multimer thereof. This is the recognition site for the protease collagenase. The adaptor would be synthesized in such a way as to provide: a 4-base overhang at the 5' end compatible with the restriction site at the 3' end of the OBP clone, a 4-base overhang at the 3' end of the adaptor to facilitate ligation to the foreign peptide coding sequence and additional bases, if needed, to ensure no frame shifts in the transition between the OBP coding sequence, the protease recognition site and the foreign peptide coding sequence. A typical arrangement for such a fusion is shown in Fig. 3. The example shown here uses an existing *Xho*1 site near the stop codon of a carrot OBP (Hatzopoulos *et al. Plant Cell*, (1990) 2:457-467). This is digested and may be ligated with an adapter constructed from the two oligonucleotides described. This adapter will form a perfect *Xho*1 overhang at an end and will not disrupt the translational frame. The other end forms an *Nco*l overhang which is arbitrarily chosen (any six-base cutter will suffice), but which encloses an ATG from the desired foreign peptide.

The final ligation product will contain an almost complete OBP gene, coding sequence for collagenase recognition motif and the desired peptide coding region all in a single reading frame. This tripartite fragment is cloned into an Agrobacterium binary plasmid (Bevan *Nucl. Acid Res.,* (1984) *12*:8711-8721) such as is widely used to transfer foreign DNA into plants (Fraley *et al. Proc. Nat'l. Acad. Sci. USA,* (1983) *80*:4803-4807) and this is used to transform oilseed plants such as rapeseed using the method of Moloney *et al. Plant Cell Rep.,* (1989) *8*:238-242) or similar procedure. Transgenic plants may be recovered from this transformation experiment and these are grown to flowering. The plants then set seed by self-fertilization.

The seeds are allowed to reach maturity (60-80 days) and then are harvested and ground in aqueous extraction buffer (Taylor *et al. Planta,* (1990) *181*:18-26). The slurry is centrifuged at 5000 xg for 20 min. and will give a surface scum. This scum is again recovered and suspended by vigorous shaking in a collagenase assay buffer (Scholtissek and Grosse, *Gene* (1988) 62:55-64). Five units of collagenase are added and the suspension is incubated with shaking for 4 h. After this time, the suspension is once again centrifuged at 5000 xg for 20 min. The surface scum is removed and the protein content of the aqueous phase is analyzed by SDS-Poly Acrylamide Gel Electrophoresis. If a band of approximately the size of the required peptide is found, the protein may be precipitated using ammonium sulfate, concentrated using ultrafiltration or lyophilized.

### B. N-terminal fusions

The hydrophilic N-terminal end of oil-body proteins permits the fusion of peptides to the N-terminal while still assuring that the foreign peptide would be retained on the outer surface of the oil body. The configuration of such fusions is shown in Fig. 2IB.

This configuration can be constructed from similar starting materials as used for C-terminal fusions, but requires the identification of a convenient restriction site close to the translational start of the oil-body protein gene. A convenient site may be created in many oil-body protein genes without any alteration in coding sequence by the introduction of a single base change just 5' to the first 'ATG'. In oil body proteins thus far studied, the second amino acid is alanine whose codon begins with a "G". The context of the sequences is shown below: 
A-C transition here yields NcoI site
↓
3′ . . . TC TCA ACA ATG GCA . . . Carrot OBP
3′ . . . CG GCA GCA ATG GCG . . . Maize 18KDa OBP
 A single base change at the adenine prior to the 'ATG' would yield in both cases . . . CCATGG . . . which is an *Nco*l site. Thus, modification of this base using the site-directed mutagenesis protocol of Kunkel (*Proc. Nat'l. Acad. Sci. USA,* (1985) *82*:488-492) will prepare this clone for use assuming no other Ncol sites in the sequence.

The coding sequence for the foreign peptide may require preparation which will allow its ligation directly into the *Nco*l site. This may typically require a single or two-base modification by site-directed mutagenesis (Kunkel, 1985, *supra*) to generate an Ncol site around the translational start of the foreign peptide. This peptide is then excised from its cloning vehicle using Ncol and a second enzyme which cuts close to the translational stop of the target. Again, using the methods described above, a second convenient site can be introduced by site-directed mutagenesis. It has been suggested by Qu and Huang (1990, *supra)* that the N-terminal methionine might be removed during processing of the protein *in vivo* and that the alanine immediately downstream of this might be acylated. To account for this possibility, it may be necessary to retain the Met-Ala sequence at the N-terminal end of the protein. This is easily accomplished using a variety of strategies which introduce a convenient restriction site into the coding sequence in or after the Ala codon. For example, by site-directed mutagenesis, the sequences could be modified as follows: 
3' ... TC TCA ACA ATG GCA GAA CGA GGC ACT TAT .....
mutate to
NarI
3' ... TC TCA ACA ATG GCA TGC CGA GGC GCC TAT .....
SphI
 This change of a single codon would introduce a *Sph*1 site into the coding sequence. A second change, which could be introduced during the same round of mutagenesis would convert two bases in codon 6 to yield GGC GCC, an *Nar*1 site. This mutated gene could then be opened with *Sph*1 and *Nar*1 to give a directional cloning cut which would eliminate three codons. Into this site could be introduced an adaptor containing a 3' overhang with the sequence CATG ... (compatible with *Sph*1) and a GC 5' overhang at the opposite end. The precise sequence of this adapter is shown below: 
Sph1 Nar1
n times
CCG CTC GGT CCG GG
CTACG GGC GAG CCA GGC CCGC
 This adapter would recreate both the *Sph*1 and *Nar*1 restriction sites which would be used for diagnostic purposes. The *Sph*1 site could now be used to open the plasmid and clone in-frame a DNA fragment enclosing the sequence for a useful peptide. Orientation of cloning could then be analyzed by cutting at any asymmetrically placed site and *Nar*1 of the plasmid.

The resultant constructs from these N-terminal fusions would be typical of the examples IB of Figure 2. They would contain an OBP promoter sequence, an in-frame fusion in the first few codons of the OBP gene of a high value peptide coding sequence with its own ATG as start signal if necessary and the remainder of the OBP gene and terminator.

This modified gene is introduced into a binary *Agrobacterium* plasmid (Bevan, (1984), supra) and mobilized into *Agrobacterium*. Transformations are performed as described above. Recovery of the high value peptide from seeds is performed as described for 'C-terminal fusions.'

### C. Internal translational fusions

A third type of fusion involves the placing of a high value peptide coding sequence internally to the coding sequence of the OBP. This type of fusion requires the same strategy as in N-terminal fusions, but may only be functional with modifications in regions of low conservation, as it is believed that regions of high conservation in these OBPs are essential for targeting of the mature protein.

The key difference in this kind of fusion is the necessity for *flanking* collagenase recognition sites for the release of the protein. This means that in place of the standard collagenase linker/adapter systems thus far described, it is necessary to have a linker with the following form: 
n times                    n times

Cohesive | CCG CTC GGT CCG | Restric- | CCG CTC GGT |
end 1 | GGC GAG CCA GGC | tion site | GGC GAG CCA GGC | end 2
 Cohesive ends 1 and 2 would be used to clone the adapter into an OBP clone in a directional manner. The nested restriction site is then used to introduce the high value peptide coding sequence flanked by appropriate restriction sites or linkers. Orientation is checked by the use of an asymmetrically placed restriction site in the high-value peptide coding sequence and one of the two restriction sites flanking the coding sequence of the collagenase recognition motif.

Mobilization of these constructs to Agrobacterium plasmids and then to plants is identical to the previously described procedure. Recovery of the high-value protein from the seeds of transgenic plants is somewhat different in that after the oil-bodies have been isolated and washed, it may be necessary to delipidate the oil-bodies in order to access the collagenase recognition sites which could be hidden inside the oil-body in the lipid phase. This step may reduce certain advantages of using oil-body proteins as carriers, but may on the other hand be very convenient for protein sequences which are labile in aqueous media or in plant cytoplasms.

### D. Inter-dimer translational fusions

It is possible to create a construct in which the entire coding sequence of the OBP is repeated. A dimeric protein produced from this construct may still contain all the necessary factors for targeting the OBP to the oil-body. Such a construct would contain a promoter region, an entire or near-complete open reading frame for an OBP but excluding the translational stop and then an entire open-reading frame of a second OBP, this time equipped with a translational 'stop' and a terminator region.

In the construction of this chimeric gene a pair of dissimilar restriction sites are either found or created at the region of the junction of the two copies. These sites are used to enable the introduction of a linker such as is described above for internal translational fusions. The linker contains not only sets of collagenase recognition motifs, but also an internal restriction site in which to nest a sequence encoding a high value protein. The form of this construct is shown in Fig. 2 III. Mobilization of this construct to *Agrobacterium* and then to plants is exactly as above. Recovery of the high value protein from seeds of the transformed plants would be performed using the same procedure as described for C-terminal fusions above.

### Example 2

### Strategy for the cloning and expression of Interleukin-1-β (IL-1-β) as a fusion with oleosins in plants

### A. Cloning and sequencing of an Arabidopsis thaliana oleosin gene

A *Brassica napus* oleosin gene (Murphy *et al,* (1991) *Biochim Biophys Acta 1088:* 86-94) was used to screen a genomic library of *A. thaliana* (cv. Columbia) in EMBL3A (Stratagene). The screening resulted in the isolation of a EMBLA3A clone (λ2.1) containing a 15 kb genomic fragment from *A. thaliana*. The oleosin was mapped within a 6.6 kb *Kpn*I insert, within this 15 kb fragment (Fig. 5). A 1.8 kb *Nco*I/*Kpn*I fragment containing the oleosin gene was end filled and subcloned in the SmaI site of RFM13mp19. The 1.8 kb insert was digested with convenient restriction enzymes and subcloned in M13mp19 for sequencing. The 1800 bp sequence of the *A. thaliana* oleosin gene is presented in Fig. la. All the cloning procedures were carried out according to Sambrook *et al.,* (1989) (Molecular Cloning: A laboratory manual 2nd ed. Cold Spring Haber Laboratory Press.)

### B. Design of an oligonucleotide encoding IL-1-β

IL-1-β consists of 9 amino acids (aa); val-gln-gly-glu-glu-ser-asn-asp-lys (Antoni et *al.,* (1986) *J. Immunol. 137*:3201-3204). The protease factor Xa can cleave a protein sequence which contains a aa sequence ile-glu-gly-arg. Cleavage takes place after the aa arg. Based on these sequences an oligonucleotide was designed (GVR11, fig. 5), which contains in addition to the IL-1-β coding sequence, the coding sequence for the factor Xa cleavage site, and 18 nucleotides of the 3' coding region of the *A. thaliana* oleosin (base position 742-759). The IL-1-β coding sequence was designed using optimal codon usage for the *B. napus* and *A. thaliana* oleosin (Table 1).

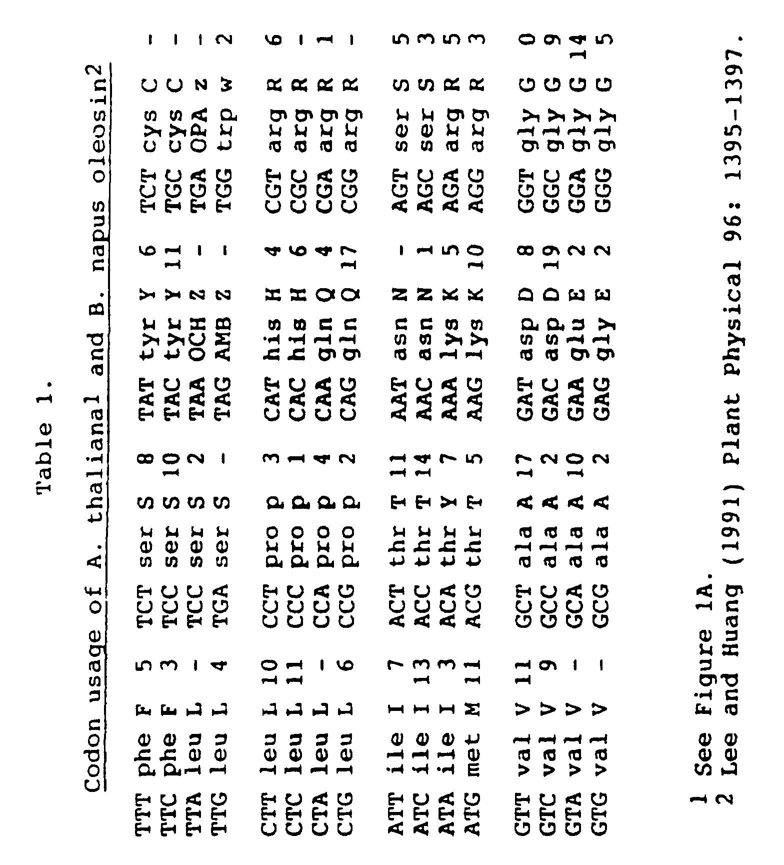

### C. Creation of an A. thaliana oleosin-IL-1-β fusion

Based on the sequence: 5′ CACACCAGGAACTCTCTGGTAAGC 3′ (base position: -838 to -814), oligonucleotide GVR10 5' CACTGCAGGAACTCTCTGGTAAGC 3' was designed. GVR10 contains a PstI restriction site (underlined) to facilitate cloning. The polymerase chain reaction (PCR) was used amplify the region between GVR10 and GVR11. The reaction mixture contained: 16 µl dNTPs (1.25 mM), 10 µl 10X PCR buffer (100 mM Tris-HCL pH 8.3, 500 mM KCL, 15 mM MgCl₂, 0.1% (w/v) gelatin), 5 µl GVR11 (20 µM) 1 µl Taq DNA polymerase (1 u/µl) and 64 µl H₂O. The reaction was carried out for 30 cycles. Each cycle consisted of 1 minute denaturing at 92°C, 1 minute annealing at 45°C and 3 minutes extension at 72°C. The PCR reaction yielded a single fragment of 1652 nucleotides.

### D. Cloning of the A. thaliana oleosin-IL-1-β (OBPIL) fusion

A 5' SalI-nopaline synthase (nos) terminator-*EcoRI* 3' sequence was isolated from pBI121 (Clontech laboratories) and cloned into the *Sa*l/*EcoR*I sites of pUC19. The plasmid was called pTerm. The 1652 bp fragment (described in C.) was isolated and digested with the restriction enzymes *Pst*I and *Sal*I. This fragment was cloned in pTerm. The resulted plasmid was called pUCOBPILT (fig. 5). This plasmid was digested with *Eco*RI and *Pst*I and resulted in the digested pUC19 vector and the *Eco*RI-A. *thaliana* oleosin-IL-1-β -nos -*Pst*I fusion *Pst*I (OBPILT). The complete sequence of OBPILT is shown in fig. 7. OBPILT was subcloned in the *Eco*RI/*Pst*I sites of pBluescript+. This plasmid (pBIOBPILT) was digested with *Pst*I and *Hind*III and the *Pst*I-OBPILT-*Hind*III fragment was subcloned in a binary *Agrobacterium* plasmid (Bin 19) (Bevan, M., (1984) *Nucl. Acid. Res.* 12: 8711-8721) containing a selection marker (neomycin phosphotransferase and *Pst*I-*Hind*III unique sites. The resulting plasmid was called pCGOBPILT. A schematic representation of the cloning procedure is shown in Fig. 5. For descriptions of various binary plasmids, see, pGA642 or 645; An *et al.* (1985) EMBO. J. 4 277-288 or pCGN1558 or 1559; MacBride and Summerfeldt (1990) *Plant. Molec. Biol.* 14 269-276.

### F. Transformation of pCGOBPILT into Agrobacterium strain EHA101

A single EHA101 colony (Hood *et al.*, (1986) *J. Bact. 168*:1291-1301) was used to inoculate 5 ml of LB+100 µg/ml kanamycin. This culture was grown for 48 hours at 28°C. This 5 ml culture was used to inoculate 500 ml of LB+100 µg/ml kanamycin. This culture was grown at 28°C until the culture reached a density of OD600=0.5 (approx. 4 hours). The cells were spun down (10 min, 5000 x g) and resuspended in 500 ml of sterile H₂O (repeated 2x). The cells were spun again and resuspended in 3 ml sterile H₂O, containing 10% glycerol. 40 µl of the cells were aliquoted in Eppendorf tubes and either directly used for electroporation, or stored at -80°C for future use. Electroporation was carried out according to Bower *et al., Nucl. Acid. Res.* (1988) 16 6127-6145. The pulse generator was set to the 25 µF capacitor, 2.5 kV and 200 ohm in parallel with the sample chamber.

### G. Transformation of Nicotiana tabacum (tobacco) with pCGOBPILT

The EHA 101 containing pCGOBPILT was used to transform tobacco leaf discs. Eight to ten centimeter long tobacco leaves were taken from greenhouse grown plants, sterilized in 70% ethanol for 20 sec. and then in 10% bleach (such as Javex ) for 8 min. The leaves were then rinsed 6 times with sterile water. The leaf edges as well as the midrib were excised from the leaves and the remaining lamina was sectioned into 5x7 mm squares or discs of 5 mm diameter. About 30 leaf discs were collected and placed into a small petri dish. The *Agrobacterium* solution was then poured over the tobacco discs and incubation occurred for 9 minutes. The leaf pieces were then blotted on sterile Whatman filter paper and placed, abaxial side down, onto media I (MS, 3% sucrose and 2 mg/l 2,4-D). Co-cultivation proceeded for the following 48 hours. At this point the leaf discs were transferred to selection media (MD, 3% sucrose, 2.5 mg/l Ba, 0.1 mg/l NAA, 500 mg/l carbenicillin, and 100 mg/l kanamycin) where they remained for the next 3-4 weeks. Once shoots began to emerge they were excised and placed onto rooting media (MS, 3% sucrose, 0.1 mg/l NAA, 500 mg/l carbenicillin, and 50 mg/l kanamycin). After sufficient roots had been formed, the tobacco plants were transferred to soil.

### H. Transformation of B. napus with pCGOBPILT

The transformation of *B. napus* was carried out according to Moloney *et al.,* (1989) *Plan Cell Rep, 8*:238-242, which disclosure is incorporated herein by reference.

### Transformation Procedure

Single colonies of *Agrobacterium tumefaciens* strain EHA 101 containing the binary plasmid were grown overnight at 28°C in AB medium. A 50µl sample of this suspension was grown overnight at 28°C in 5 ml of MG/L broth supplemented with appropriate antibiotics. This bacterial suspension was pelleted by centrifugation for 15 min. at 10,000 x g then resuspended in 10 ml of MS medium containing 3% sucrose and at pH 5.8. A thin film of this suspension was used to cover the base of a 5 cm petri dish. Individual excised cotyledons were taken from the plates described above and the cut surface of their petioles was immersed into this bacterial suspension for a few seconds. They were immediately returned to the same MS plates from which they had been taken. The cotyledons were co-cultivated with the *Agrobacterium* for 72 h. No feeder layers were employed.

After co-cultivation, the cotyledons were transferred to regeneration medium comprising MS medium supplemented with 20µM benzyladenine, 3% sucrose, 0.7% phytagar, pH 5.8 and 500 mg/l carbenicillin (Pyopen, Ayerst) and 15 mg/l kanamycin sulphate (Boehringer-Mannheim). Again the petioles were carfully embedded in the agar to a depth of 2 mm. Plating density was maintained at 10 explants per plate. Higher densities reduce regeneration frequency.

### Selection and Plant Regeneration

The explants were maintained on regeneration medium under light and temperature conditions specified above for 2-3 weeks. During this time many shoots appeared on over half the explants with relatively little callus formation. Some of these shoots undergo bleaching by the fourth week of culture. The remaining green shoots were subcultured onto shoot elongation medium which was the same as regeneration medium minus the benzyladenine. One or two weeks on this medium permitted the establishment of apical dominance from the shoot clusters formed. The shoots so derived were transferred to "rooting" medium containing MS medium, 3% sucrose, 2mg/l indole butyric acid, 0.7% phytagar and 500 mg/l carbenicillin. No kanamycin was used at this stage as it was found that more rapid root establishment occurred without the selection agent while very few "escapes" actually succeeded in rooting after the two rounds of selection on regeneration and shoot elongation medium.

### I. Stable integration of OBPILT in the tobacco and B. napus genomes

Putative transformed plants were tested for neomycin phosphotransferase activity. Genomic DNA from plants showing this activity was isolated. Southern blotting was performed in order to demonstrate that the sequences between the T-DNA borders (OBPILT and neomycin phosphotransferase gene) were stably integrated in to the genomes of *B. napus* and tobacco. The tobacco Southern was probed with the *A. thaliana* oleosin gene, and the neomycin phosphotransferase gene. The *B. napus* Southern was probed with the neomycin phosphotransferase gene.

### J. Expression of the oleosin-IL-1-β fusion in tobacco plants

RNA was isolated from developing embryos obtained from transformed and untransformed plants. Northern blotting was carried out using the *A. thaliana* oleosin as a gene probe. In all the tested transformed plants a 850 nt transcript could be detected. The size of these transcripts correspond to the expected size of the oleosin-IL-1-β mRNA. These transcripts could not be detected in the untransformed plants.

### K. Accumulation of the oleosin-IL-1-β protein

Oil-body proteins were isolated from transformed tobacco seeds (Holbrook *et al.,* (1991) *Plant Physical* 97:1051-1058. PAGE was performed and the protein were transferred from the gel to PVDF membranes. An antibody, which was raised against a 22 kDa oleosin of *B. napus,* was used to detect the oleosin-IL-1-β fusion in the tobacco seeds. This antibody recognizes all the major oleosins in *B. napus* and *A. thaliana.* In addition, this antibody recognizes the tobacco oleosins. Tobacco oleosins have different sizes from the *A. thaliana* and *B. napus* oleosins. In the transformed tobacco seeds the anti-22 kDa antibody recognized a 20 kDa-protein, which was not present in the untransformed tobacco seed. The predicted size of the oleosin-IL-1-β fusion is 20.1 kDa. A summary of the results is shown in Table 2.

By expressing a peptide of interest conjugated to an oil body protein, or a sufficient portion thereof to provide for getting to the oil bodies, the peptide of interest can be easily purified so as to be substantially free of other cellular components. The fusion protein can be cleaved following purification or may be used without cleavage. The subject methods and compositions provide a fast, simple method for purifying a polypeptide of interest.

All publications and patent applications mentioned in this specification are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

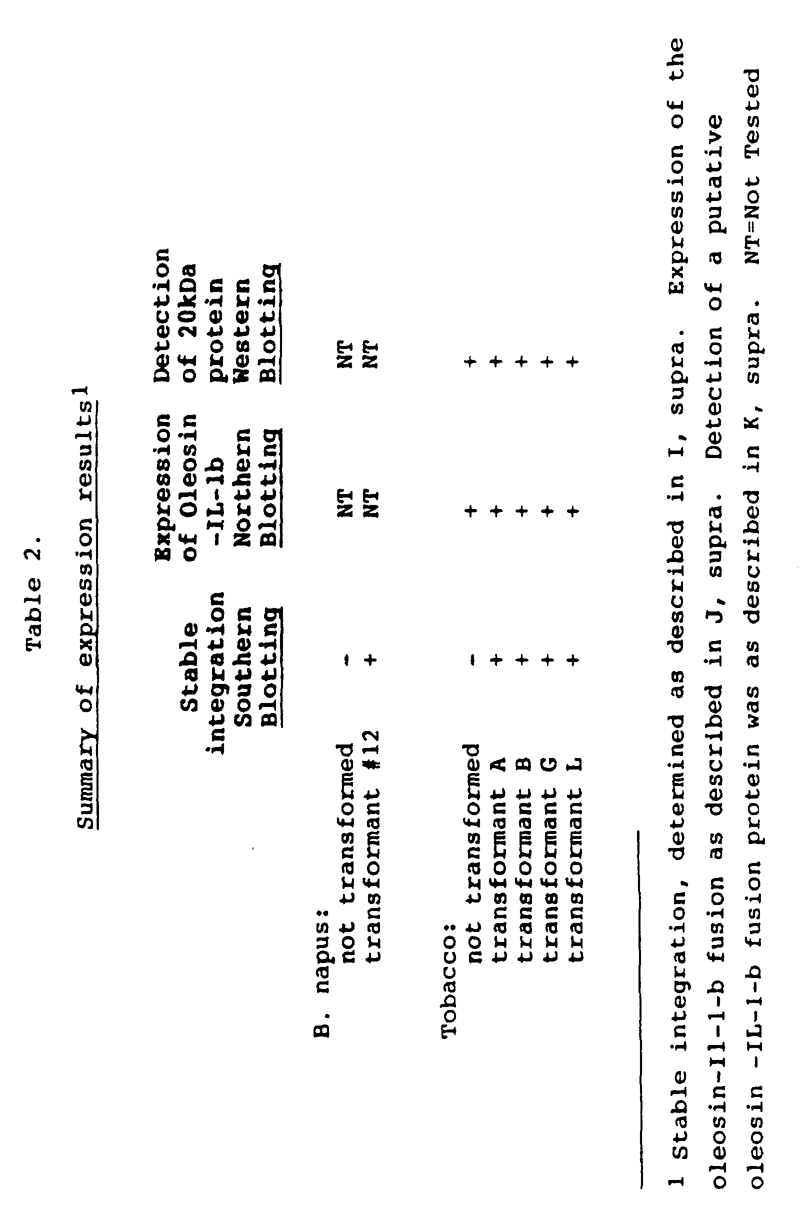

## Claims

1. A chimeric DNA construct encoding a chimeric polypeptide and comprising:
(a) a first DNA sequence encoding at least the central hydrophobic core of an oil body protein and sufficient to provide for targeting of the fusion polypeptide to an oil body and
(b) a second DNA sequence encoding a peptide wherein said peptide is other than a polypeptide naturally present in an oil body.

2. The chimeric DNA construct according to claim 1, further comprising:
vector DNA containing at least one regulatory sequence operatively associated with said chimeric DNA sequence which is capable of directing replication of said chimeric DNA in a host cell.

3. The chimeric DNA construct according to claim 2 wherein said regulatory sequence is further capable of directing expression of said chimeric DNA in a host cell.

4. The chimeric DNA construct according to any one of claims 1 to 3, wherein said DNA is cDNA.

5. An expression cassette comprising as components, in the direction of transcription:
- a regulatory DNA sequence comprising a sufficient portion of the region 5' to the translational start site of a gene expressed in seed to provide for expression of a DNA sequence in seed;
- a chimeric DNA sequence encoding a chimeric polypeptide and comprising:
(a) a first DNA sequence encoding at least the central hydrophobic region of an oil body protein and sufficient to provide for targeting of the chimeric polypeptide to an oil body, said first DNA sequence including at least one restriction site, and
(b) a second DNA sequence encoding a peptide wherein said peptide is other than a polypeptide naturally present in an oil body; and
- a translational and transcriptional termination region;
wherein said components are operably linked and expressed by said chimeric DNA sequence which is regulated by said regulatory DNA sequence.

6. The expression cassette according to claim 5, wherein said regulatory DNA sequence is from a gene expressed in a cereal or grain seed cell.

7. The expression cassette according to claim 5 or claim 6 wherein said regulatory DNA sequence is of an oil body protein gene.

8. The expression cassette according to claim 5, wherein at least one of said regulatory DNA sequence and said first DNA sequence comes from the genome of Arabidopsis thaliana.

9. The expression cassette according to any one of claims 5-8, wherein said expression cassette includes at least one restriction site between just 5' to the codon for the initiating methionine and 5' to the translational stop signal of said oil body protein gene.

10. The expression cassette according to claim 9 wherein said restriction site is a synthetic restriction site.

11. The expression cassette according to any one of claims 5-10, further comprising an oligonucleotide adapter coding for a protease recognition site inserted into said restriction site.

12. The expression cassette according to claim 11, wherein said protease is collagenase.

13. A method for obtaining expression of a peptide of interest in seed, said method comprising:
transforming a host plant cell with an expression cassette according to any one of claims 5-12 or a DNA construct according to any one of claims 1-4 under genomic integration conditions.

14. A method according to claim 13 comprising growing said plant to produce seed whereby said polypeptide of interest is expressed as a chimeric protein with the expression product of said oil body protein gene.

15. The method according to claim 14, further comprising: isolating said chimeric protein from oil bodies in cells of said seed.

16. The method according to claim 15, wherein said isolating comprises:
lysing cells of said seed to release said oil bodies; and
disrupting said oil bodies whereby said chimeric protein is released.

17. The method according to claim I S or claim 16, wherein said isolating further comprises:
contacting said chimeric protein with a protease capable of recognising a protease recognition site in said chimeric protein located prior to the N-terminus of said peptide of interest.

18. The method according to claim 17, further comprising:
prior to said contacting, binding said chimeric protein to a solid support comprising an antibody capable of binding to the expression product of said oil body protein gene.

19. A method according to any one of claims 13-18 which method comprises purifying said peptide of interest.

20. The method according to any one of claims 13-19, wherein said peptide of interest is other than a peptide encoded by a plant genome.

21. A seed, a plant or a plant cell comprising an expression cassette according to any one of claims 5-12 or a DNA construct according to any one of claims 1-4.

22. A chimeric polypeptide comprising at least the central hydrophobic core of an oil body protein and sufficient to provide for targeting to an oil body and a second polypeptide, said chimeric polypeptide being expressed by the expression cassette according to any one of claims 5-12 or a chimeric DNA construct according to any one of claims 1-4.

## Patentansprüche

1. Chimäres DNA-Konstrukt, welches ein chimäres Polypeptid codiert und folgendes umfaßt:
(a) eine erste DNA-Sequenz, welche wenigstens den zentralen hydrophoben Kern eines Ölkörperproteins codiert und ausreicht, ein Zielen des Fusionspolypeptids auf einen Ölkörper bereitzustellen, und
(b) eine zweite DNA-Sequenz, welche ein Peptid codiert, wobei das Peptid von einem Polypeptid, das natürlicherweise in einem Ölkörper vorhanden ist, verschieden ist.

2. Chimäres DNA-Konstrukt nach Anspruch 1, welches weiterhin folgendes umfaßt:
Vektor-DNA, welche wenigstens eine regulatorische Sequenz enthält, die funktionsfähig mit der chimären DNA-Sequenz verbunden ist und welche in der Lage ist, die Replikation der chimären DNA in einer Wirtszelle zu steuern.

3. Chimäres DNA-Konstrukt nach Anspruch 2, wobei die regulatorische Sequenz weiterhin in der Lage ist, die Expression der chimären DNA in einer Wirtszelle zu steuern.

4. Chimäres DNA-Konstrukt nach einem der Ansprüche 1 bis 3, wobei die DNA cDNA ist.

5. Expressionskassette, die in der Richtung der Transkription als Bestandteile folgendes umfaßt:
- eine regulatorische DNA-Sequenz, die einen ausreichenden Abschnitt der Region 5' zu der Translationsstartstelle eines Gens umfaßt, das in Samen exprimiert wird, zur Bereitstellung von Expression einer DNA-Sequenz in Samen;
- eine chimäre DNA-Sequenz, welche ein chimäres Polypeptid codiert und folgendes umfaßt:
(a) eine erste DNA-Sequenz, welche wenigstens den zentralen hydrophoben Bereich eines Ölkörperproteins codiert und ausreicht, ein Zielen des chimären Polypeptids auf einen Ölkörper bereitzustellen, wobei die erste DNA-Sequenz wenigstens eine Restriktionsschnittstelle umfaßt, und
(b) eine zweite DNA-Sequenz, welche ein Peptid codiert, wobei das Peptid von einem in einem Ölkörper natürlicherweise vorhandenen Polypeptid verschieden ist, und
- eine Translations- und Transkriptionsterminierungsregion,
wobei die Bestandteile funktionsfähig miteinander verknüpft sind und von der chimären DNA-Sequenz, welche von der regulatorischen DNA-Sequenz gesteuert wird, exprimiert werden.

6. Expressionskassette nach Anspruch 5, wobei die regulatorische DNA-Sequenz von einem Gen stammt, das in einer Getreide- oder Komsamenzelle exprimiert wird.

7. Expressionskassette nach Anspruch 5 oder Anspruch 6, wobei die regulatorische DNA-Sequenz vom Gen eines Ölkörperproteins stammt.

8. Expressionskassette nach Anspruch 5, wobei wenigstens eine von der regulatorischen DNA-Sequenz und der ersten DNA-Sequenz von dem Genom von Arabidopsis thaliana stammt.

9. Expressionskassette nach einem der Ansprüche 5 bis 8, wobei die Expressionskassette wenigstens eine Restriktionsschnittstelle zwischen gerade 5' zu dem Codon für das initialisierende Methionin und 5' zu dem Stopsignal der Translation des Gens für Ölkörperprotein umfaßt.

10. Expressionskassette nach Anspruch 9, wobei die Restriktionsschnittstelle eine synthetische Restriktionsschnittstelle ist.

11. Expressionskassette nach einem der Ansprüche 5 bis 10, welche weiterhin einen Oligonukleotidadapter aufweist, der eine Proteaseerkennungsstelle codiert, die in die Restriktionsschnittstelle eingefügt ist.

12. Expressionskassette nach Anspruch 11, wobei die Protease Collagenase ist.

13. Verfahren zum Erreichen von Expression eines interessierenden Peptids in Samen, wobei das Verfahren folgendes umfaßt:
Transformieren einer Wirtspflanzenzelle mit einer Expressionskassette nach einem der Ansprüche 5 bis 12 oder einem DNA-Konstrukt nach einem der Ansprüche 1 bis 4 unter Bedingungen für genomische Integration.

14. Verfahren nach Anspruch 13, welches Züchten der Pflanze zur Produktion von Samen umfaßt, wobei das interessierende Polypeptid als ein chimäres Protein mit dem Expressionsprodukt des Gens für Ölkörperprotein exprimiert wird.

15. Verfahren nach Anspruch 14, welches weiterhin folgendes umfaßt: Isolieren des chimären Proteins aus Ölkörpern in Zellen des Samens.

16. Verfahren nach Anspruch 15, wobei das Isolieren folgendes umfaßt:
Lysieren von Zellen des Samens zur Freisetzung der Ölkörper und
Zerstören der Ölkörper, wobei das chimäre Protein freigesetzt wird.

17. Verfahren nach Anspruch 15 oder Anspruch 16, wobei das Isolieren weiterhin folgendes umfaßt:
Inkontaktbringen des chimären Proteins mit einer Protease, die in der Lage ist, eine Proteaseerkennungsstelle in dem chimären Protein, welche vor dem N-Terminus des interessierenden Proteins angeordnet ist, zu erkennen.

18. Verfahren nach Anspruch 17, welches weiterhin folgendes umfaßt:
Binden des chimären Proteins an einen festen Träger, welcher einen Antikörper aufweist, der in der Lage ist, an das Expressionsprodukt des Gens für Ölkörperprotein zu binden, vor dem Inkontaktbringen.

19. Verfahren nach einem der Ansprüche 13 bis 18, wobei das Verfahren Reinigung des interessierenden Peptids umfaßt.

20. Verfahren nach einem der Ansprüche 13 bis 19, wobei das interessierende Peptid von einem Peptid, das von einem Pflanzengenom codiert wird, verschieden ist.

21. Samen, Pflanze oder Pflanzenzelle mit einer Expressionskassette nach einem der Ansprüche 5 bis 12 oder einem DNA-Konstrukt nach einem der Ansprüche 1 bis 4.

22. Chimäres Polypeptid, welches wenigstens den zentralen hydrophoben Kem eines Ölkörperproteins aufweist und ausreicht, ein Zielen auf einen Ölkörper bereitzustellen, und ein zweites Polypeptid, wobei das chimäre Polypeptid von der Expressionskassette nach einem der Ansprüche 5 bis 12 oder einem chimären DNA-Konstrukt nach einem der Ansprüche 1 bis 4 exprimiert wird.

## Revendications

1. Produit d'assemblage d'ADN chimère codant pour un polypeptide chimère et comprenant :
(a) une première séquence d'ADN codant pour au moins le noyau central hydrophobe d'une protéine de corps huileux et suffisante pour assurer le ciblage du polypeptide de fusion à un corps huileux, et
(b) une seconde séquence d'ADN codant pour un peptide, ledit peptide étant autre qu'un polypeptide naturellement présent dans un corps huileux.

2. Produit d'assemblage d'ADN chimère suivant la revendication 1, comprenant en outre :
un ADN de vecteur contenant au moins une séquence régulatrice associée de manière fonctionnelle à ladite séquence d'ADN chimère qui est capable de diriger la réplication dudit ADN chimère dans une cellule hôte.

3. Produit d'assemblage d'ADN chimère suivant la revendication 2, dans lequel ladite séquence régulatrice est en outre capable de diriger l'expression dudit ADN chimère dans une cellule hôte.

4. Produit d'assemblage d'ADN chimère suivant l'une quelconque des revendications 1 à 3, dans lequel ledit ADN est un ADNc.

5. Cassette d'expression comprenant, comme constituants, dans le sens de transcription :
- une séquence d'ADN régulatrice comprenant une partie suffisante de la région 5' au site d'initiation de traduction d'un gène exprimé dans des graines pour assurer l'expression d'une séquence d'ADN dans des graines ;
- une séquence d'ADN chimère codant pour un polypeptide chimère et comprenant :
(a) une première séquence d'ADN codant pour au moins la région centrale hydrophobe d'une protéine de corps huileux et suffisante pour assurer le ciblage du polypeptide chimère à un corps huileux, ladite première séquence d'ADN comprenant au moins un site de restriction, et
(b) une seconde séquence d'ADN codant pour un peptide, ledit peptide étant autre qu'un polypeptide présent naturellement dans un corps huileux ; et
- une région de terminaison de traduction et de transcription ;
lesdits constituants étant liés de manière fonctionnelle et exprimés par ladite séquence d'ADN chimère qui est régulée par ladite séquence d'ADN régulatrice.

6. Cassette d'expression suivant la revendication 5, dans laquelle ladite séquence d'ADN régulatrice provient d'un gène exprimé dans une cellule de graines de céréales ou de plantes donnant des grains.

7. Cassette d'expression suivant la revendication 5 ou la revendication 6, dans laquelle ladite séquence d'ADN régulatrice provient d'un gène de protéine de corps huileux.

8. Cassette d'expression suivant la revendication 5, dans laquelle au moins une desdites séquences consistant en ladite séquence d'ADN régulatrice et ladite première séquence d'ADN provient du génome d'Arabidopsis thaliana.

9. Cassette d'expression suivant l'une quelconque des revendications 5 à 8, ladite cassette d'expression comprenant au moins un site de restriction entre la région juste en 5' au codon pour la méthionine initiatrice et la région en 5' au signal de terminaison de traduction dudit gène de protéine de corps huileux.

10. Cassette d'expression suivant la revendication 9, dans laquelle ledit site de restriction est un site de restriction synthétique.

11. Cassette d'expression suivant l'une quelconque des revendications 5 à 10, comprenant en outre un adaptateur oligonucléotidique codant pour un site de reconnaissance de protéase inséré dans ledit site de restriction.

12. Cassette d'expression suivant la revendication 11, dans laquelle ladite protéase est la collagénase.

13. Procédé pour parvenir à l'expression d'un peptide intéressant dans des graines, ledit procédé comprenant :
la transformation d'une cellule d'une plante hôte avec une cassette d'expression suivant l'une quelconque des revendications 5 à 12 ou un produit d'assemblage d'ADN suivant l'une quelconque des revendications 1 à 4 dans des conditions d'intégration génomique.

14. Procédé suivant la revendication 13, comprenant en outre la culture de ladite plante pour produire des graines, ledit polypeptide intéressant étant ainsi exprimé sous forme d'une protéine chimère avec le produit d'expression dudit gène de protéine de corps huileux.

15. Procédé suivant la revendication 14, comprenant en outre : l'isolement de ladite protéine chimère des corps huileux dans les cellules desdites graines.

16. Procédé suivant la revendication 15, dans lequel ledit isolement comprend :
la lyse des cellules desdites graines pour libérer lesdits corps huileux ; et
la rupture desdits corps huileux, ce qui provoque la libération de ladite protéine chimère.

17. Procédé suivant la revendication 15 ou la revendication 16, dans lequel ledit isolement comprend en outre :
la mise en contact de ladite protéine chimère avec une protéase capable de reconnaître un site de reconnaissance de protéase dans ladite protéine chimère situé avant l'extrémité N-terminale dudit peptide intéressant.

18. Procédé suivant la revendication 17, comprenant en outre :
avant ladite mise en contact, la liaison de ladite protéine chimère à un support solide comprenant un anticorps capable de se lier au produit d'expression dudit gène de protéine de corps huileux.

19. Procédé suivant l'une quelconque des revendications 13 à 18, qui comprend la purification dudit peptide intéressant.

20. Procédé suivant l'une quelconque des revendications 13 à 19, dans lequel ledit peptide intéressant est autre qu'un peptide codé par un génome végétal.

21. Graine, plante ou cellule végétale comprenant une cassette d'expression suivant l'une quelconque des revendications 5 à 12 ou un produit d'assemblage d'ADN suivant l'une quelconque des revendications 1 à 4.

22. Polypeptide chimère comprenant au moins le noyau central hydrophobe d'une protéine de corps huileux et suffisant pour assurer le ciblage à un corps huileux et un second polypeptide, ledit polypeptide chimère étant exprimé par la cassette d'expression suivant l'une quelconque des revendications 5 à 12 ou un produit d'assemblage d'ADN chimère suivant l'une quelconque des revendications 1 à 4.
